# EUROPÄISCHE PATENTSCHRIFT

(11) **EP 0 998 239 B1**
(45) Veröffentlichungstag und Bekanntmachung des Hinweises auf die Patenterteilung: **06.10.2004**
(21) Anmeldenummer: 98938697.4
(22) Anmeldetag: 21.07.1998
(51) Int. Cl.: A61B 19/00

(54) **ENDOSKOPISCHES INSTRUMENT ZUR DURCHFÜHRUNG VON ENDOSKOPISCHEN EINGRIFFEN ODER UNTERSUCHUNGEN UND ENDOSKOPISCHES INSTRUMENTARIUM, ENTHALTEND EIN SOLCHES ENDOSKOPISCHES INSTRUMENT**
ENDOSCOPIC INSTRUMENT FOR CARRYING OUT ENDOSCOPIC INTERVENTIONS OR EXAMINATIONS AND ENDOSCOPIC INSTRUMENTATION CONTAINING SUCH AN ENDOSCOPIC INSTRUMENT
INSTRUMENT ENDOSCOPIQUE POUR INTERVENTIONS OU EXAMENS ENDOSCOPIQUES ET APPAREILLAGE ENDOSCOPIQUE RENFERMANT UN TEL INSTRUMENT

(30) Priorität: 24.07.1997 DE 19731894
(43) Veröffentlichungstag der Anmeldung: 10.05.2000
(73) Patentinhaber: Karl Storz GmbH & Co. KG, 78532 Tuttlingen (DE)
(72) Erfinder: IRION, Klaus, M., D-78576 Liptingen (DE)
(74) Vertreter: Weller, Wolfgang, Dr.
(86) Internationale Anmeldenummer: PCT/EP1998/004575
(87) Internationale Veröffentlichungsnummer: WO 1999/004719

(56) Entgegenhaltungen:
- WO-A-97/24988
- US-A- 3 840 015
- US-A- 5 002 561
- US-A- 5 350 391

## Beschreibung

Die Erfindung betrifft ein endoskopisches Instrument zur Durchführung von endoskopischen Untersuchungen oder Eingriffen, mit einem Schaft, mit einem am proximalen Ende des Schaftes angeordneten Griff und mit zumindest einem am distalen Ende des Schaftes angeordneten Arbeitselement, wobei am distalen Endbereich des Instruments zumindest eine Markierung vorgesehen ist, die einen Stoff aufweist, der über eine Lichtquelle anregbar ist.

Die Erfindung betrifft ferner ein endoskopisches Instrumentarium, das ein solches endoskopisches Instrument enthält und ferner eine Lichtzuleitungsvorrichtung und ein endoskopisches Beobachtungsinstrument, insbesondere ein Endoskop, das mit einer Lichtquelle verbunden ist.

Ein endoskopisches Instrument der eingangs genannten Art ist aus der US-A-5 350 39A1 bekannt.

Der über eine Lichtquelle anregbare Stoff besteht in Form von farbigen Streifen, die über eine Lichtquelle zur Lumineszenz anregbar sind.

Aus der US-A-3 840 015 ist es bekannt, chirurgische Instrumente wie Skalpelle, Nadeln oder dgl. mit einer photolumineszierenden Substanz zu versehen, damit das Instrument nach einer Strahlung mit Anregungslicht während einer Operation erkannt werden kann.

Aus der WO-A-97/24988 ist ein flexibles chirurgisches Instrument bekannt, das mit einer Markierung am Ende eines Schaftes versehen ist.

Derartige endoskopische Instrumente und derartige endoskopische Instrumentarien sind allgemein bekannt und werden in der immer weiter verbreiteten minimal-invasiven Chirurgie eingesetzt.

Zunehmend werden in der Endoskopie zur Erkennung und Behandlung von Gewebeveränderungen Verfahren der photodynamischen Diagnose (PDD) und Therapie (PDT) eingesetzt. Aus dem Sonderdruck "Endo World, URO Nr. 17/1-D, 1997, "Photodynamische Diagnose (PDD) zur Früherkennung des Harnblasenkarzinoms" der Karl Storz GmbH & Co., Tuttlingen, Deutschland, ist ein System bekannt, um in Verbindung mit einem sich tumorspezifisch anreichernden Photosensibilisator, der unter speziellem Anregungslicht eine Fluoreszenz zeigt, Tumore bzw. maligne Gewebeveränderungen zu erkennen. Als Prekursor für einen solchen Photosensibilisator wird z.B. Aminolävulinsäure (ALA) erfolgreich eingesetzt.

Aus der US 5 408 996 ist es bekannt, malignes Gewebe endoskopisch darzustellen, indem ein dem Gewebe zugeführter Markierungsstoff zur Fluoreszenz angeregt wird.

Ein Erkennen von Gewebeveränderungen ist auch auf der Basis der sogenannten Autofluoreszenz, ausgelöst durch oberflächlich in äußeren Gewebeschichten vorkommende natürliche Fluoreszenzstoffe, möglich.

Bei all diesen Verfahren wird Licht (sogenanntes Anregungslicht) eines speziellen Wellenlängenbereiches in den den Fluoreszenzstoff enthaltenden Gewebebereich eingekoppelt, und dieser Fluoreszenzstoff wird dadurch zur Fluoreszenz angeregt. Der Fluoreszenzwellenlängenbereich ist gegenüber dem Anregungswellenbereich stets langwelliger. Bei der Verwendung von ALA wird im Blauen angeregt (380 bis 430 nm), und die Fluoreszenz erfolgt im Roten (635 nm). Aus dem eingangs erwähnten Prospekt "Endo Wörld" ist auf Seite 5 beeindruckend zu erkennen, wie gegenüber normalem Licht, sogenanntem Weißlicht, bei einer Fluoreszenz-Anregung ein Tumor klar definiert ersichtlich gemacht werden kann. Selbst winzige Satellitentumore eines fluoreszierenden papillären Tumors können klar abgesetzt von dem Haupttumor erkannt und entsprechend behandelt bzw. durch einen chirurgischen endoskopischen Eingriff entfernt werden.

Bei Autofluoreszenzuntersuchungen kann die Anregung auch im Blauen erfolgen, die Fluoreszenz zeigt sich primär im grünen und roten Spektralbereich. Auch eine Anregung im UV-Bereich kann vorgenommen werden.

Da die Fluoreszenzintensität weit unter der Anregungsintensität liegt, wird zur Erkennung der Fluoreszenzemission der Spektralbereich des Anregungslichts im empfangenden Beobachtungssystem durch Filter fast nahezu abgeblockt. Nur die fluoreszierenden Areale sind deutlich erkennbar.

Dies hat nun nachteiligerweise zur Folge, daß bei der Einführung von Instrumenten in den vom Endoskop ausgeleuchteten und aufgenommenen Bereich diese Instrumente im Fluoreszenzbetrieb des Beleuchtungssystems schlecht oder gar nicht zu erkennen sind. Daher muß bislang andauernd vom Fluoreszenzbetrieb auf den Weißlichtbetrieb und umgekehrt umgeschaltet werden, um ein Instrument gezielt an den zu untersuchenden oder zu behandelnden Gewebebereich heranzuführen. Dies ist umständlich und für den Operateur äußerst lästig, störend und vor allem auch gefährlich bzw. kritisch für den Eingriff.

Diese Instrumente können vielfältiger Natur sein. Es können Instrumente sein, die bei Untersuchungen (Diagnose) oder auch bei chirurgischen Eingriffen (Therapie) eingesetzt werden.

Aus der DE 39 33 199 C2 ist es grundsätzlich bekannt, das distale Endstück eines flexiblen Endoskopes mit einer Markierung zu versehen, die es ermöglicht, das Endstück bei röntgenologischen Untersuchungen darzustellen.

Es ist Aufgabe der vorliegenden Erfindung, ein endoskopisches Instrument zu schaffen, das auch bei Fluoreszenzbetrieb deutlich zu erkennen ist, bzw. ein endoskopisches Instrumentarium zu schaffen, mit dem endoskopische chirurgische Eingriffe oder Untersuchungen unter Fluoreszenzbedingungen durchgeführt werden können.

Erfindungsgemäß wird die Aufgabe bei einem endoskopischen Instrument dadurch gelöst, daß der Stoff zur Fluoreszenz anregbar ist, und daß der fluoreszierende Stoff derart ausgewählt ist, daß dessen Anregungsbereich im Anregungsbereich einer Gewebeautofluoreszenz oder im Anregungsbereich eines tumorspezifischen Photosensibilisators liegt.

Wird der fluoreszierende Stoff derart ausgewählt, daß dessen Anregungsbereich im Anregungsbereich einer Gewebeautofluoreszenz liegt, hat dies den erheblichen Vorteil, da die Gewebeautofluoreszenz nur äußerst schwach ist, daß nicht mit einer anderen, diese Autofluoreszenz störenden Anregungsfrequenz gearbeitet wird, sondern genau in demselben Anregungsbereich, so daß dann auch die entsprechende Fluoreszenz wie die Autofluoreszenz erzielt wird.

Die Auswahl des Anregungsbereichs im Anregungsbereich eines tumorspezifischen Photosensibilisators hat allgemein den Vorteil, daß nur mit einem ganz bestimmten Anregungsbereich angeregt wird und dabei sowohl beim Tumor als auch dann beim markierten Instrument eine Fluoreszenz hervorgerufen wird. Gegenseitige Störeinflüsse beim Anregen des tumorspezifischen Photosensibilisators einerseits und des fluoreszierenden Stoffes der Markierung andererseits sind dann nicht mehr möglich und somit systemimmanent ausgeschlossen. Der Wellenlängenbereich von 370 nm bis 440 nm ist ein Anregungsbereich, mit dem der zur Markierung von Tumoren einsetzbare Prekursor ALA, der in das photosensible Protoporphyrin IX als Photosensibilisator umgesetzt wird, angeregt werden kann.

Unter Lichtquelle im Sinne der vorliegenden Erfindung wird eine Strahlungsquelle verstanden, die Licht im UV-, im sichtbaren und/oder im IR-Bereich abstrahlt. Licht in diesem Wellenlängenbereich ist, im Gegensatz zu Röntgen-Licht, für das Gewebe des zu untersuchenden oder zu behandelnden Körpers oder für den Beobachter nicht beeinträchtigend oder gar schädlich, so daß auch langandauernde Operationen, oder aus Sicht des Operateurs zahlreiche operative Eingriffe im Fluoreszenzbetrieb ohne Strahlungsschäden durchgeführt werden können.

Es kann nunmehr unter Fluoreszenzbetrieb ein Instrument aufgrund der fluoreszierenden Markierung klar erkannt werden und außerdem dessen Relativlage zum zu untersuchenden oder zu entferndenden Gewebe eindeutig bestimmt werden. Dadurch kann unter fluoreszenzdiagnostischen Lichtverhältnissen auf einfache Weise z.B. eine Biopsiezange oder ein anderes Instrument, z.B. eine Laserfaser, unter endoskopischer Beobachtung zum fluoreszierenden Tumorareal geführt werden und z.B. eine Gewebeprobe entnommen werden, ohne daß auf die endoskopische Standard-Weißlichtbeleuchtung umgeschaltet werden muß. Es kann also während des Eingriffes dauernd im Fluoreszenzbetrieb gearbeitet werden, bei dem sowohl der zu behandelnde Tumor als auch das dazu eingesetzte Instrument bzw. dessen Arbeitselemente klar und deutlich erkennbar sind. Dadurch wird nun die Möglichkeit geschaffen, sowohl die photodynamische Diagnose (PDD) als auch die photodynamische Therapie (PDT) für den Operateur unter gleichbleibenden Lichtbedingungen, also ohne ein Umschalten zwischen Weißlicht und Fluoreszenzbetrieb durchzuführen.

Bei dem endoskopischen Instrumentarium ist die Lichtquelle derart ausgewählt, daß der fluoreszierende Stoff am endoskopischen Instrument dadurch anregbar ist.

Es kann vorgesehen sein, daß sowohl der fluoreszierende Stoff auf dem endoskopischen Instrument als auch der fluoreszierende Stoff im Gewebe durch ein und dieselbe Lichtquelle anregbar ist und jeweils dieselbe farbige Fluoreszenz zeigt, es kann auch vorgesehen sein, dies mit unterschiedlichen Wellenlängen zu betreiben und dementsprechend unterschiedliche Fluoreszenzphänomene zu erzielen. Der fluoreszierende Stoff kann direkt auf das Instrument aufgetragen oder in dieses inkorporiert werden. Er ist selbstverständlich immer an einer solchen Stelle angebracht, die im Beobachtungsbereich eines Endoskopes liegt.

Somit wird die Aufgabe vollkommen gelöst.

In einer weiteren Ausgestaltung der Erfindung ist die Markierung als am Instrument angebrachtes Markierungselement ausgebildet.

Diese Maßnahme hat den Vorteil, daß das endoskopische Instrument wie gewohnt hergestellt und anschließend, falls dieses Instrument für den Fluoreszenzbetrieb vorgesehen ist, kann das Markierungselement mit dem gewünschten fluoreszierenden Stoff angebracht werden.

In einer weiteren Ausgestaltung der Erfindung ist das Markierungselement lösbar angebracht.

Diese Maßnahme hat den Vorteil, daß bspw. zu Reinigungszwecken oder falls das endoskopische Instrument auch im Nicht-Fluoreszenzbetrieb eingesetzt werden soll, das Markierungselement abgenommen werden kann. Dadurch ist auch die Möglich keit eröffnet, bei unterschiedlichen Arten von Operationen, oder wenn unterschiedliche Gewebe mit unterschiedlichen Photosensibilisatoren versetzt werden, entsprechend ausgewählte oder geeignete Markierungselemente an dem endoskopischen Instrument anzubringen.

In einer weiteren Ausgestaltung der Erfindung ist das zumindest eine distale Arbeitselement mit einer Markierung versehen.

Diese Maßnahme hat nun den erheblichen Vorteil, daß durch Vorsehen der Markierung am Arbeitselement der Operateur besonders exakt erkennen kann, an welcher Stelle das Arbeitselement sich gerade befindet, bspw. um einen chirurgischen Eingriff exakt anzusetzen und durchzuführen.

In einer weiteren Ausgestaltung, bei der die Arbeitselemente als zwei Maulteile ausgebildet sind, ist vorgesehen, diese jeweils mit einer Markierung zu versehen.

Diese Maßnahme hat nun den erheblichen Vorteil für den Operateur, daß er bspw. das abzutrennende Gewebeteil exakt zwischen die beiden gespreizten Maulteile bringen kann, er somit genau die Position der gespreizten Maulteile relativ zu dem abzutrennenden, ebenfalls markierten Gewebeteil bringen kann und dann an einer exakt vorbestimmten und zutreffenden Stelle das Gewebe abtrennen kann.

In einer weiteren Ausgestaltung der Erfindung ist sowohl das Arbeitselement als auch der proximale Endbereich des Schaftes je mit einer Markierung versehen.

Diese Maßnahme hat nicht nur den Vorteil für den Operateur, daß er die Relativlage des Schaftes zu den Arbeitselementen, z.B. Maulteile, feststellen kann, sondern diese Ausgestaltung kann auch besonders günstig für ein sogenanntes "Instrumententracking" herangezogen werden. Bei einem Instrumententracking soll das endoskopische Aufnahmesystem (Endoskop, Kamera) bzw. das endoskopische Bild jeweils dem manipulierenden Instrument folgen. Aus der DE 195 29 950 Cl ist bekannt, ein Instrumententracking aufgrund von großflächigen Farbmarkierungen auf Instrumenten durchzuführen. Da aber im Körper selbst alle möglichen Farben des sichtbaren Bereichs vorkommen können, ist eine eindeutige Zuordnung einer vom Endoskop erfaßten Farbinformation zu dem Instrument nicht möglich.

Derzeit führt ein assistierender Operateur das Endoskop mit proximalseitiger Kamera nach. Dies ist mit dem Nachteil verbunden, daß bei langwierigen Operationen aufgrund von Ermüdungserscheinungen des Assistenten das manuelle Nachführen des Endoskopes nicht mehr mit der ausreichenden Genauigkeit durchgeführt wird, so daß für den Operateur kurzzeitig das Operationsfeld zeitweilig nicht mehr erkennbar ist. Aufgrund von Ermüdungserscheinungen kann das Endoskop hin- und herbewegt werden, so daß eine Bildqualitätsminderung durch Wackeln erscheint. In dieser Ausgestaltung ist mit dem Instrumentarium keine sogenannte "Solochirurgie" durchzuführen. Durch das Vorsehen von mehreren Markierungen oder Markierungen, die sich über einen größeren Flächenbereich erstrecken, ist nun die Möglichkeit geschaffen, eine automatische Führung über die Fluoreszenzmarkierungen durchzuführen, da die jeweils dreidimensionale Lage apparativ exakt bestimmt werden kann. Wird nun das Instrument mit den Markierungen von dem Operateur in seiner Lage verändert, kann das Bilderfassungssystem diese Lageänderung erkennen, und entsprechend kann dann das Beobachtungssystem, bspw. das Endoskop, entsprechend nachgeführt, also ein "tracking" durchgeführt werden. So können bspw. externe 3 D-Sensoren, die die Position des Instruments fortlaufend erfassen, dazu herangezogen werden, um über eine Endoskopsteuereinrichtung den Arbeitsbereich der Instrumente automatisch immer im endoskopischen Bild zum Liegen zu bringen.

In einer weiteren Ausgestaltung der Erfindung ist das Markierungselement als auf einen rohrförmigen Schaft aufschiebbare Schlauchtülle ausgebildet.

Diese Maßnahme hat den Vorteil, daß das Markierungselement einfach auf den Schaft aufgeschoben werden kann. Hat diese Schlauchtülle schon eine gewisse geometrische Ausdehnung, können mehrere Bestimmungspunkte, bspw. deren Anfang und deren Ende, schon für eine räumliche Lagepositionierung gegenüber einem fixen Bezugspunkt herangezogen werden.

In einer weiteren Ausgestaltung der Erfindung weist das Markierungselement eine Beschichtung aus transparentem Glas oder eine transparente Kunststoffbeschichtung auf, die den fluoreszierenden Stoff bedeckt.

Diese Maßnahme hat den Vorteil, daß der eigentlich fluoreszierende Stoff hermetisch vom Operationsfeld abgeriegelt ist, dennoch über die transparente Beschichtung zur Fluoreszenz angeregt werden kann. Dadurch können dann auch fluoreszierende Stoffe herangezogen werden, die an sich nicht mit Körperflüssigkeiten in Verbindung treten sollen, sei es weil sie dadurch in deren Fluoreszenzeigenschaften verändert werden oder sei es weil diese Stoffe schädlich für das Gewebe sein könnten. Dadurch wird auch die Bandbreite der im medizinischen Bereich einsetzbaren fluoreszierenden Stoffe wesentlich verbreitert.

In einer weiteren Ausgestaltung der Erfindung ist das Markierungselement derart ausgebildet, daß es im Körper, an dem der endoskopische Eingriff durchgeführt wird, ablegbar und dort verankerbar ist.

Diese Maßnahme hat nun den Vorteil, daß bspw. bei einem Diagnoseschritt schon eine Markierung zurückgelassen bzw. verankert wird, so daß bei einem anschließenden operativen Eingriff die Stelle sofort wieder erkannt werden kann. Beim operativen Eingriff, der möglicherweise von einem anderen Operationsteam durchgeführt wird als der Diagnoseschritt, kann die speziell geformte Marke durch Fluoreszehzanregung sofort erkannt werden, und demzufolge auch das zu behandelnde Gewebe, das mit dieser Marke markiert wurde.

In einer weiteren Ausgestaltung der Erfindung ist der fluoreszierende Stoff ausgewählt aus der Gruppe bestehend aus Fluoreszin, Acridinorange, den Tetracyclinen, Eosin, Cadmiumsulfid, Aminolävulinsäure, Aminolävulinsäurehydrochloriden, Porphyrine, Rhodamin B, Rhodamin G, Auramin, Auramin-Karbolfuchsin, Nilblausulfat.

Dies sind gängige fluoreszierende Stoffe, sogenannte Fluorochrome.

In einer weiteren Ausgestaltung der Erfindung ist der fluoreszierende Stoff derart ausgewählt, daß er im Wellenlängenbereich von 200 nm bis 900 nm zur Fluoreszenz anregbar ist.

Dieser Wellenlängenbereich, der sich beidseits über den Wellenlängenbereich des sichtbaren Lichtes von 400 nm bis 750 nm hinaus erstreckt, eröffnet nun einen breiten Anwendungsbereich. So kann bspw. im nicht sichtbaren UV-Bereich angeregt werden und eine Fluoreszenz im sichtbaren Bereich erzielt werden. Diese Maßnahme hat bspw. den Vorteil, daß das Anregungslicht für den Operateur nicht zu erkennen und nicht störend ist, sondern er lediglich die Fluoreszenz im sichtbaren Bereich erkennt.

Dadurch ist auch weiter die Möglichkeit eröffnet, bspw. im nicht sichtbaren UV-Bereich anzuregen und eine Fluoreszenz im ebenfalls nicht sichtbaren Infrarotbereich zu erzielen. Dies wird dann vorteilhaft sein, wenn bspw. automatisch kontrollierte Vorgänge stattfinden, bspw. ein Instrumententracking, das ohne Beeinflussung des Auges des Operateurs stattfinden kann.

In einer solchen Ausgestaltung der Erfindung wird der fluoreszierende Stoff bspw. so ausgewählt, daß er im Wellenlängenbereich von 320 bis 380 nm, also von nicht sichtbaren UV-Licht zur Fluoreszenz anregbar ist, die dann im sichtbaren Bereich erfolgen kann.

In einer weiteren Ausgestaltung der Erfindung sind mehrere Markierungen mit unterschiedlich anregbaren fluoreszierenden Stoffen vorgesehen.

Diese Maßnahme hat den erheblichen Vorteil, daß ein solches Instrument flexibel bei unterschiedlichen diagnostischen oder operativen Eingriffen bzw. in Zusammenhang mit unterschiedlichen tumorspezifischen Photosensibilisatoren eingesetzt werden kann, da durch das Vorhandensein der unterschiedlich anregbaren fluoreszierenden Stoffe dann jeweils zumindest ein Stoff vorliegt, der bei dem speziellen Einsatzzweck anregbar ist.

Diese Möglichkeit kann auch vorteilhafterweise bei dem "Instrumententracking" eingesetzt werden, so können bspw. eine oder mehrere Markierungen, die außerhalb des sichtbaren Bereiches anregbar sind und ggf. außerhalb des sichtbaren Bereiches fluoreszieren, zum Tracking herangezogen werden, eine weitere Markierung als eine für den Operateur sichtbare Markierung.

In einer weiteren Ausgestaltung der Erfindung sind mehrere Markierungen vorgesehen, die unterschiedliche Konzentrationen an fluoreszierendem Stoff enthalten.

Diese Maßnahme hat den Vorteil, daß, je nach den örtlichen Gegebenheiten und der Fluoreszenzintensität des Tumors, entsprechend abgeglichen gearbeitet werden kann, also mit hoher oder geringer Intensität.

Eine solche Markierung mit unterschiedlich anregbaren fluoreszierenden Stoffen ermöglicht auch beim Einstellen eines endoskopischen Instrumentariums, z.B. mit einem Endoskop und einer Videokamera, eine optimale Bestrahlungsstärke durch die Anregungslichtquelle zu Beginn der Operation einzustellen.

In einer weiteren Ausgestaltung der Erfindung weist die Markierung einen solchen fluoreszierenden Stoff auf, der dem eines im Körper abgelegten Markierungselementes entspricht. Diese Maßnahme hat in Zusammenhang mit der zuvor erwähnten Maßnahme des im Körper abgelegten Markierungselementes den Vorteil, daß mit ein und derselben Anregung gearbeitet werden kann, so daß sowohl das im Körper bereits abgelegte Markierungselement als auch das mit der entsprechenden Markierung versehene endoskopische Instrument vom Operateur deutlich erkennbar sind, und bspw. auch das abgelegte Element einfach wieder mit dem medizinischen Instrument ergriffen und entfernt werden kann.

Bei dem endoskopischen Instrumentarium, das neben dem endoskopischen Instrument noch eine Lichtzuleitvorrichtung und ein endoskopisches Beobachtungsinstrument, insbesondere ein Endoskop, das mit einer Lichtquelle verbunden ist, aufweist, ist von weiterem Vorteil, daß zumindest ein endoskopisches Manipulationsinstrument, insbesondere ein Trokar, vorgesehen ist, durch den das Beobachtungsinstrument in den Körper einführbar ist, wobei an der Innenseite des Manipulationsinstruments zumindest eine Markierung mit einem fluoreszierenden Stoff entsprechend dem endoskopischen Instrument vorgesehen ist.

Diese Maßnahme hat den erheblichen Vorteil, daß die Funktion eines FDD- oder PDT-Systems in vivo anhand einer intrakorporalen Referenz getestet werden kann. Wird das mit dem fluoreszierenden Stoff beschichtete Instrument in den Trokar eingeschoben, kann über die an der Innenseite oberflächlich verteilten fluoreszierenden Stoffe die Funktionsfähigkeit des Systems überprüft werden. Darüber hinaus ist es schon möglich, verschiedene Parameter, wie bspw. die ausreichende Leistungsdichte des Anregungslichtes, die spektrale Zusammensetzung des Anregungslichtes für den Einsatzfall optimal einzustellen. Zusätzlich kann ein optimaler Farbabgleich für eine das endoskopische Bild erfassende Kamera erfolgen.

Bei einem solchen endoskopischen Instrumentarium ist weiter von Vorteil, daß das Beobachtungsinstrument ein Endoskop ist, das mit einer endoskopischen Kamera versehen ist.

Diese Maßnahme hat den Vorteil, daß der Operateur nicht direkt am Endoskop arbeiten muß, sondern dieses Bild über eine endoskopische Kamera aufgenommen wird, so daß dann die Möglichkeit besteht, diese Bildinformation zu speichern, bspw. um bei späteren Untersuchungen Unterschiede festzustellen oder diese Bildinformation zur besseren Aufbereitung einem Bildverarbeitungssystem zuzuführen. So können bspw. Informationen, die bei einem Diagnoseschritt erzielt worden sind, zu Beginn einer Operation mit den dabei erfaßten Daten verglichen werden, und bspw. ein Fortschreiten des Tumors oder ggf. auch ein Abnehmen aufgrund anderweitiger chemischer Behandlungen festgestellt werden. Dies erlaubt auch, die Abnahme der Fluoreszenz während einer PDT-Behandlung zu erfassen (Dosimetrie bei der PDT), bspw. anhand im Körper verbliebener markierter Elemente.

In einer weiteren Ausgestaltung der Erfindung ist der endoskopischen Kamera ein Bildverarbeitungssystem nachgestaltet, das die fluoreszierenden Markierungen im endoskopischen Bild laufend detektiert.

Diese Maßnahme hat nun den Vorteil, daß aufgrund des Bildverarbeitungssystems über geeignete Filter störende oder nicht erwünschte Fluoreszenzerscheinungen unterdrückt oder schwache Signale verstärkt werden können, so daß dem Operateur ein optimales Bild sowohl des Gewebes als auch des markierten Instruments dargeboten wird.

In einer weiteren Ausgestaltung der Erfindung gibt die Lichtquelle zumindest im spektralen Anregungsbereich des fluoreszierenden Stoffes gepulstes Licht ab, wobei die Pulsfrequenz der Videobildfrequenz bzw. der Videohalbbildfrequenz der endoskopischen Kamera entspricht.

Diese Maßnahme hat den Vorteil, daß ein exaktes Instrumententracking mit dieser Pulstechnik durchgeführt werden kann und auch kleinste Lageveränderungen, die vom menschlichen Auge nicht erfaßt werden können, bereits erfaßt werden können. Der PAL-Standard beträgt 25 bzw. 50 Hz, der NTSC-Standard 30 bzw. 60 Hz. Die Pulstechnik erlaubt ein Instrumententracking, ohne daß dadurch das menschliche Auge beeinflußt bzw. gestört wird, da bei den in der Videotechnik üblichen Bildfrequenzen das menschliche Auge zu träge ist, um Unterschiede wahrzunehmen.

In einer weiteren Ausgestaltung des Instrumentariums ist vorgesehen, daß das Beobachtungsinstrument distalseitig ein transparentes Element mit einem fluoreszierenden Stoff aufweist.

Diese Ausgestaltung hat den erheblichen Vorteil, daß vom Beobachtungsinstrument aufgenommenes Reflexionslicht, das im nicht sichtbaren Bereich liegt, durch den fluoreszierenden Stoff, der von diesem Bereich angeregt wird, in ein sichtbares Fluoreszenzphänomen umgewandelt werden kann.

Es versteht sich, daß die vorstehend genannten und die nachstehend noch zu erläuternden Merkmale nicht nur in den angegebenen Kombinationen sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels näher beschrieben und erläutert.

Es zeigen:
- Fig. 1: eine stark schematisierte Ansicht eines endoskopischen Instrumtariums während eines endoskopischen Eingriffes, und
- Fig. 2: eine stark vergrößerte perspektivische ausschnittsweise Ansicht des distalen Endbereichs des endoskopischen Instruments, das bei dieser Operation eingesetzt wird.

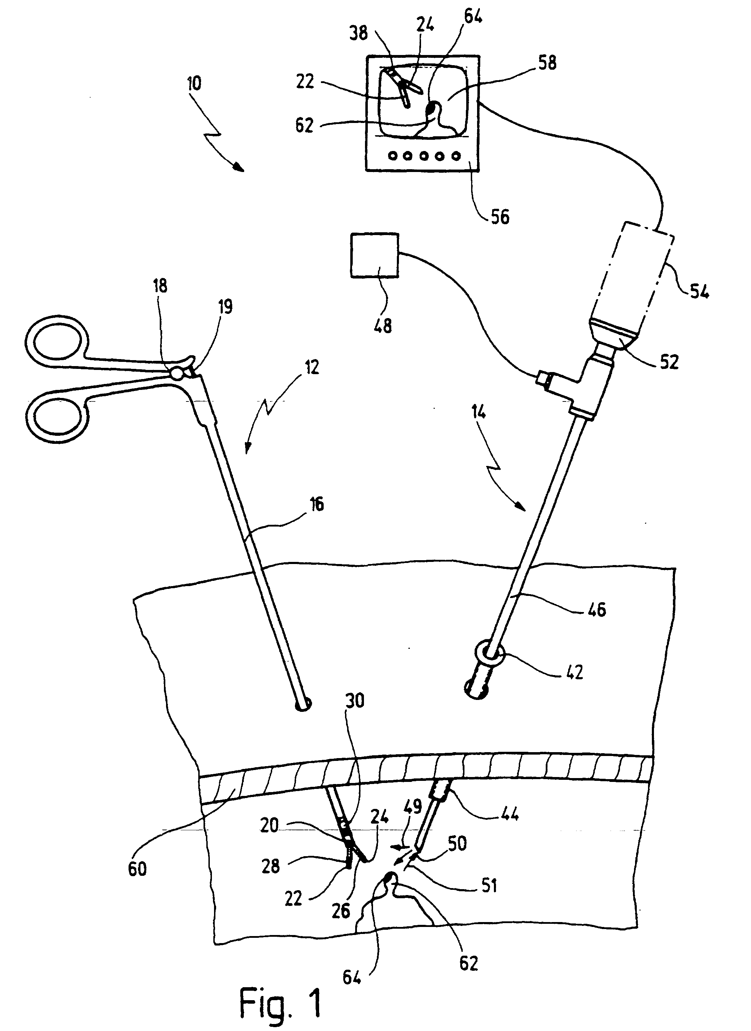

In Fig. 1 ist ein endoskopisches Instrumentarium in seiner Gesamtheit mit der Bezugsziffer 10 versehen.

Das endoskopische Instrumentarium 10 weist ein endoskopisches Instrument 12 sowie ein Endoskop 14 auf.

Das endoskopische Instrument 12 ist eine Faßzange, die einen langerstreckten rohrförmigen Schaft 16 aufweist. Am proximalen Ende ist der Schaft 16 mit einem Griff versehen, der aus zwei scherenartigen Griffelementen besteht, die über ein Scharnier miteinander verbunden sind. Am distalen Ende ist ein Arbeitselement 20 in Form von zwei Maulteilen 22 und 24 angeordnet.

Die Maulteile 22 und 24 stehen über ein Betätigungselement 19 mit dem Griff in Verbindung, so daß ein Verschwenken des beweglichen Griffelementes eine lineare Bewegung des Betätigungselementes 19 längs des Schaftes 16 verursacht und, je nach Bewegungsrichtung, die Maulteile 22 und 24 gespreizt bzw. geschlossen werden.

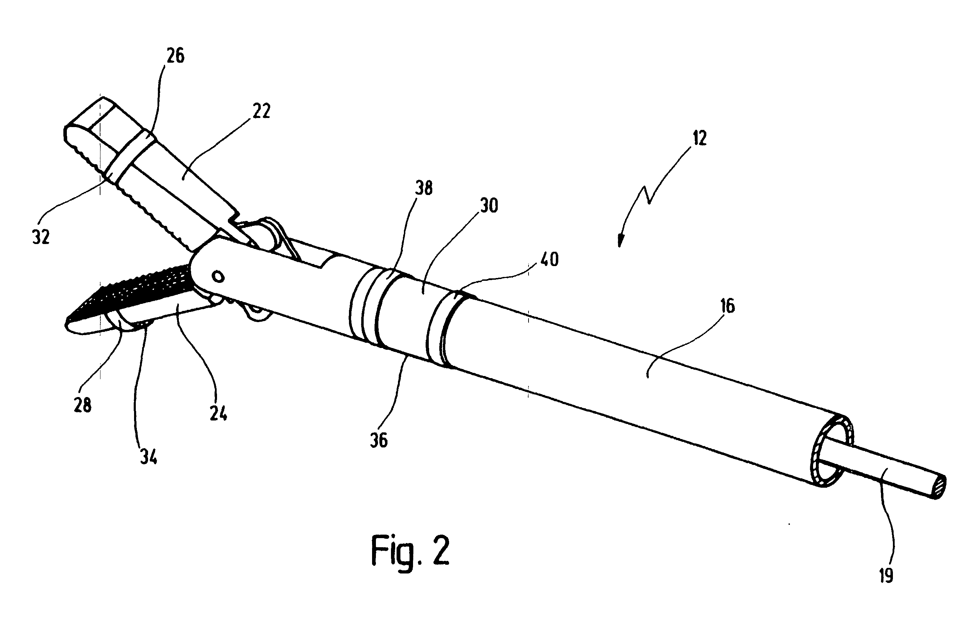

Wie aus der vergrößerten ausschnittsweisen Darstellung von Fig. 2 zu erkennen, ist jedes Maulteil 22 bzw. 24 mit einer Markierung 26 bzw. 28 versehen. Ferner ist im Bereich des distalen Endes des Schaftes 16 eine weitere Markierung 30 vorgesehen.

Die Markierung 26 und 28 der Maulteile 22 und 24 besteht aus separat ansetzbaren Markierungselementen 32 und 34.

Die Markierungselemente 32 und 34 sind halbbogenförmig ausgebildet und können von der Außenseite auf die Maulteile 22 und 24 fest haftend aufgeklipst werden.

Jedes Markierungselement 32 und 34 besteht aus einer transparenten Kunststoffdoppelfolie, zwischen der ein fluoreszierender Stoff eingelagert ist. Unter fluoreszierenden Stoff wird ein Stoff verstanden, der nach Anregung mit Licht eines bestimmten Wellenlängenbereiches Fluoreszenz zeigt. Der Wellenlängenbereich des Anregungslichtes liegt im Bereich von 200 nm bis 900 nm, also umfaßt den Bereich des sichtbaren Lichtes, etwa 400 nm bis 750 nm und auch den nicht sichtbaren UV- und IR-Bereich.

Die Markierung 30 besteht aus einer Schlauchtülle 36, die auf die Außenseite des Schaftes 16 aufgeschoben ist und an ihren gegenüberliegenden Enden jeweils einen Markierungsring 38 und 40 aufweist.

Die Markierungsringe 38 und 40 beinhalten ebenfalls einen fluoreszierenden Stoff, der gleich wie der fluoreszierende Stoff der Markierungselemente 32 und 34 sein kann, oder auch ein anderer fluoreszierender Stoff sein kann, bspw. ein Stoff, der im UV-Bereich angeregt werden kann und im IR-Bereich Fluoreszenz zeigt.

Das Endoskop 14 weist einen Endoskopschaft 46 auf, der durch ein Manipulationsinstrument 42 in Form eines Trokars 44 durch eine Bauchdecke 60 eines menschlichen Körpers in eine Körperhöhle eingebracht ist.

Proximal ist der Endoskopschaft 46 über einen seitlichen Ansatz mit einer Lichtquelle 48 verbunden. Die Lichtquelle 48 kann ultraviolettes (UV-), sichtbares und/oder infrarotes (IR-) Licht ausstrahlen, wobei auch ganz bestimmte und auch mehrere definierte Wellenlängenbereiche ausgestrahlt werden können. Im Endoskopschaft 46 sind Lichtleiter in Form eines Glasfaserbündels enthalten, die das von der Lichtquelle 48 ausgehende Licht am distalen Ende des Endoskopschaftes 46 abgeben, wie das durch Pfeile 49 angedeutet ist.

Im Endoskop 14 ist ferner eine Optik 50 aufgenommen, die aus einem im Endoskopschaft 46 angeordneten Stablinsensystem besteht, das am proximalen Ende in einem mit einer Okularmuschel 52 versehenen Okular endet. Somit kann am distalen Ende des Endoskopschaftes 46 auftreffendes Fluoreszenzlicht, wie das durch einen Pfeil 51 angedeutet ist, über die Optik an das distale Ende des Endoskopes 14 geführt werden.

Soll an einem im Körper befindlichen Gewebe 62 bspw. eine Gewebeprobe entnommen werden, werden sowohl das endoskopische Instrument 12 als auch das Endoskop 14, wie in der minimal-invasiven Chirurgietechnik bekannt, über kleine Körperöffnungen z.B. durch die Bauchdecke 16 eingebracht. Diese Körperöffnungen werden üblicherweise durch Trokare bzw. deren Trokardorne bewerkstelligt. Nach Einführen sowohl des endoskopischen Instrumentes 12 als auch des Endoskopes 14 kann der Operateur sein Auge an die Okularmuschel 52 legen und die im Bauchraum unterhalb der Bauchdecke 16 ersichtlichen Bereiche erfassen. Bei der eingangs beschriebenen photodynamischen Diagnose (PDD) wurde dem Patienten zuvor, z.B. Aminolävulinsäure (ALA), verabreicht. Befindet sich im Bereich des untersuchten Gewebes 62 ein Tumor 64, so reichert sich in diesem ALA and wird in das photosensible Protoporphyrin IX umgesetzt und dieser fluoreszierende Stoff kann durch ein entsprechendes Anregungslicht, hier im Blauen (380 nm bis 430 nm), über einen speziellen Umwandlungsprozeß zur Fluoreszenz angeregt werden, die im Roten (635 nm) erfolgt. Wie eingangs beschrieben, ist die Lichtquelle 48 so ausgestaltet, daß sie entweder Weißlicht oder speziell Licht im Bereich der Anregung von ALA, also blaues Licht von 380 nm bis 430 nm aussendet. Würde ein medizinisches Instrument aus Medizinerstahl bestehen, wie das bei endskopischen Instrumenten 12 in Form einer Faßzange zur Entnahme einer Gewebeprobe üblich ist, würde diese Zange kaum zu erkennen sein. Aufgrund der Tatsache, daß nun das endoskopische Instrument 12 mit Markierungen 26, 28 und 30 versehen ist, werden auch diese Markierungen zur Fluoreszenz angeregt. Im einfachsten Fall ist die Markierung so ausgewählt, daß sie einen fluoreszierenden Stoff enthält, der ebenfalls mit blauem Licht angeregt werden kann und im roten Licht Fluoreszenzerscheinungen zeigt. Der Operateur kann somit über die Optik 50 des Endoskops 14 sowohl das fluoreszierende Gewebe 62 als auch die fluoreszierenden Markierungen 26, 28 bzw. ggf. auch 30 des endoskopischen Instruments 12 erkennen und somit exakt die Gewebeprobe mittels des endoskopischen Intruments 12 entnehmen. Bei einem chirurgischen Eingriff kann das endoskopische Instrument 12 als Schneidezange ausgebildet sein, die bspw. ein von einem Tumor 64 befallenden Gewebebereich abtrennt.

In Fig. 1 ist mit der strichpunktierten Linie eine endoskopische Kamera 54 angedeutet, die auf die Okularmuschel 52 des Endoskopes 14 aufgesetzt ist. Die endoskopische Kamera 54 steht mit einem Bildverarbeitungssystem 56 in Verbindung, das auf einem Monitor ein Videobild 58 erzeugt.

In diesem Fall kann der Operateur den Operationsbereich über den Monitor verfolgen. Aus dem Videobild 58 ist zu erkennen, daß bei der zuvor genannten Anregungsfrequenz, bei der der Photosensibilisator ALA durch Umwandlung in Photoporphyrin Fluoreszenz zeigt, diese verstärkt im Bereich eines Tumors 64 am Gewebe 62 stattfindet. Auf dem Videobild 58 ist auch der distale Endbereich des endoskopischen Instrumentes 12 zu erkennen, zumindest die Markierungen der Maulteile 22 und 24. Somit kann ganz gezielt bspw. eine Gewebeprobe im Bereich des Tumors 64 entnommen werden.

Durch das Zwischenschalten eines Bildverarbeitungssystems 56 kann ein Instrumententracking durchgeführt werden, d.h. das Endoskop 14 kann über einen hier nicht näher dargestellten Stellantrieb nachgestellt werden. Dazu strahlt die Lichtquelle 48 pulsierendes Licht aus, das spezifisch die Markierung 38 und auch ggf. die Markierungen 26 und 28 an den Maulteilen 22 und 24 zur Fluoreszenz anregt. Durch diese drei Ortsparameter ist die räumliche Lage des endoskopischen Instrumentes 12 relativ zum Endoskop 14 durch externe 3 D-Sensoren erfaßbar und kann mit dem Stellantrieb für das Endoskop gekoppelt sein. Wird nun die Lage des endoskopischen Instrumentes 12 verändert, erfaßt dies das Bildverarbeitungssystem 56 und gibt ein entsprechendes Signal an den Stellantrieb, der dann das Endoskop 14 in eine solche Lage bringt, daß von dessen optischem Aufnahmebereich am distalen Ende wieder das distale Ende des endoskopischen Instrumentes 12 erfaßt wird. Nähert sich also bspw. der Operateur mit dem endoskopischen Instrument 12 dem Tumor 64 des Gewebes 62, so ist die Steuerung derart, daß immer die Maulteile 22 und 24 bzw. die Markierung 38 am distalen Endbereich des Schaftes 38 im optischen Blickfeld der Optik 50 ist.

An der Innenseite der Trokarhülse 44 sind Markierungen mit zahlreichen fluoreszierenden Stoffen enthalten, bspw. auch die fluoreszierenden Stoffe, die auf den Maulteilen 22 und 24 bzw. der Markierung 38 vorhanden sind, ggf. ist auch Protoporphyrin als Referenzsubstanz enthalten. Wird das distale Ende des Endoskopschaftes 46 durch den Trokar 14 hindurchgeschoben, so kann, noch bevor das distale Ende in die Körperhröhle eingearnngen ist, die Lichtquelle 48 und das Bildverarbeitungssystem 56 in Betrieb gesetzt werden, um festzustellen, ob das System arbeitet, und es kann zugleich ein Bildabgleich vorgenommen werden. Erfolgt die Pulsation z.B. mit der Videofrequenz des Kamerasystems (PAL: 50 Hz, NTSC: 60 Hz), so kann die markierte Stelle sehr einfach über die Referenzbildung der Farbwerte aufeinanderfolgende Halbbilder bestimmt werden. Aufgrund der relativ hohen Frequenz kann ein menschlicher Betrachter die Farbunterschiede zeitlich nicht auflösen und daher den störenden Unterschied nicht erkennen. Ähnliches gilt bei einer Anregung im sichtbaren und einer spezifischen Fluoreszenz im IR-Bereich.

Durch mit Fluoreszenzstoffen markierte Instrumente kann über eine Erfassung über mindestens zwei räumlich zugeordnete Bildaufnehmer eine Positionserkennung, d.h. eine Lokalisation der räumlichen Instrumentenkoordination bzw. Richtung erfolgen. Dadurch, daß diese Stellen mit wellenlängenmäßig unterschiedlich abstrahlenden fluoreszierenden Stoffen markiert sind und/oder flächenmäßig in unterschiedlichen Ausdehnungen markiert sind, kommt es bei den erfassenden Bildaufnehmer-Chips zu keinen Mehrdeutigkeiten der Zuordnung. Somit ist auch die Möglichkeit eröffnet, daß das Bildverarbeitungssystem 56 nicht nur erkennt, wo sich das endoskopische Instrument 12 befindet, sondern ob auch das zutreffende Instrument eingesetzt wird.

## Patentansprüche

1. Endoskopisches Instrument zur Durchführung von endoskopischen Untersuchungen oder Eingriffen, mit einem Schaft (16), mit einem am proximalen Ende des Schaftes (16) angeordneten Griff (18) und mit zumindest einem am distalen Ende des Schaftes (16) angeordneten Arbeitselement (20), wobei am distalen Endbereich des Instrumentes (12) zumindest eine Markierung (26, 28, 30) vorgesehen ist, die einen Stoff aufweist, der über eine Lichtquelle (48) anregbar ist, **dadurch gekennzeichnet, daß** der Stoff zur Fluoreszenz anregbar ist, und daß der fluoreszierende Stoff derart ausgewählt ist, daß dessen Anregungsbereich im Anregungsbereich einer Gewebeautofluoreszenz oder im Anregungsbereich eines tumorspezifischen Photosensibilisators liegt.

2. Endoskopisches Instrument nach Anspruch 1, **dadurch gekennzeichnet, daß** die Markierung (26, 28, 30) als am Instrument (12) angebrachtes Markierungselement (32, 34, 36) ausgebildet ist.

3. Endoskopisches Instrument nach Ansprüch 2, **dadurch gekennzeichnet, daß** das Markierungselement (32, 34, 36) lösbar angebracht ist.

4. Endoskopisches Instrument nach einem der Ansprüche 1 bis 3, **dadurch gekennzeichnet, daß** das zumindest eine distale Arbeitselement (20) mit einer Markierung (28, 30) versehen ist.

5. Endoskopisches Instrument nach Anspruch 4, **dadurch gekennzeichnet, daß** die Arbeitselemente (20) als zwei Maulteile (22, 24) ausgebildet sind, die jeweils mit einer Markierung (28, 30) versehen sind.

6. Endoskopisches Instrument nach einem der Ansprüche 1 bis 5, **dadurch gekennzeichnet, daß** sowohl am zumindest einen Arbeitselement (20) als auch im distalen Endbereich des Schaftes (16) je eine Markierung (26, 28, 30) vorgesehen ist.

7. Endoskopisches Instrument nach Anspruch 6, **dadurch gekennzeichnet, daß** das Markierungselement (30) als auf einen rohrförmigen Schaft (16) aufschiebbare Schlauchtülle (36) ausgebildet ist.

8. Endoskopisches Instrument nach einem der Ansprüche 2 bis 7, **dadurch gekennzeichnet, daß** das Markierungselement eine Beschichtung aus transparentem Glas oder transparentem Kunststoff aufweist, die den fluoreszierenden Stoff bedeckt.

9. Endoskopisches Instrument nach Anspruch 2 oder 3, **dadurch gekennzeichnet, daß** das Markierungselement im Körper, an dem der endoskopische Eingriff durchgeführt wird, ablegbar und dort verankerbar ist.

10. Endoskopisches Instrument nach einem der Ansprüche 1 bis 9, **dadurch gekennzeichnet, daß** der fluoreszierende Stoff ausgewählt ist aus der Gruppe bestehend aus Fluoreszin, Eosin, den Porphyrinen, Cadmiumsulfid, Aminolävulinsäure, Aminolävulinsäurehydrochlorid, Acridinorange, Tetracycline, Auramin, Rhodamin B, Rhodamin G, Auramin-Karbolfuchsin, Nilblausulfat.

11. Endoskopisches Instrument nach einem der Ansprüche 1 bis 10, **dadurch gekennzeichnet, daß** der fluoreszierende Stoff derart ausgewählt ist, daß er im Wellenlängenbereich von 200 nm bis 900 nm zur Fluoreszenz anregbar ist.

12. Endoskopisches Instrument nach Anspruch 11, **dadurch gekennzeichnet, daß** der fluoreszierende Stoff derart ausgewählt ist, daß er im Wellenlängenbereich von 320 nm bis 380 nm anregbar ist.

13. Endoskopisches Instrument nach Anspruch 1, **dadurch gekennzeichnet, daß** der fluoreszierende Stoff im Bereich von 400 nm bis 500 nm, dem Anregungsbereich einer Gewebeautofluoreszenz, zur Fluoreszenz anregbar ist.

14. Endoskopisches Instrument nach Anspruch 1, **dadurch gekennzeichnet, daß** der fluoreszierende Stoff im Bereich von 370 nm bis 440 nm, dem Anregungsbereich eines tumorspezifischen Photosensibilisators, anregbar ist.

15. Endoskopisches Instrument nach einem der Ansprüche 1 bis 14, **dadurch gekennzeichnet, daß** mehrere Markierungen (26, 28, 30) mit unterschiedlich anregbaren fluoreszierenden Stoffen vorgesehen sind.

16. Endoskopisches Instrument nach einem der Ansprüche 1 bis 15, **dadurch gekennzeichnet, daß** mehrere Markierungen (26, 28, 30) vorgesehen sind, die unterschiedliche Konzentrationen an fluoreszierenden Stoffen enthalten.

17. Endoskopisches Instrument nach Anspruch 9, **dadurch gekennzeichnet, daß** die Markierung (26, 28, 30) einen solchen fluoreszierenden Stoff aufweist, der dem eines im Körper abgelegten Markierungselement entspricht.

18. Endoskopisches Instrumentarium, enthaltend ein endoskopisches Instrument (12) nach einem der Ansprüche 1 bis 17, und ferner enthaltend eine Lichtzuleitungsvorrichtung und ein endoskopisches Beobachtungsinstrument, das mit einer Lichtquelle (48) verbunden ist, die derart ausgewählt ist, daß der fluoreszierende Stoff zur Fluoreszenz anregbar ist.

19. Endoskopisches Instrumentarium nach Anspruch 18, **dadurch gekennzeichnet, daß** das endoskopische Instrumentarium ein Endoskop (14) ist.

20. Endoskopisches Instrumentarium nach Anspruch 18 oder 19, **dadurch gekennzeichnet, daß** zumindest ein endoskopisches Manipulationsinstrument, vorgesehen ist, durch den das Beobachtungsinstrument in den Körper einführbar ist, und daß an der Innenseite des Manipulationsinstrumentes zumindest eine Markierung mit einem fluoreszierenden Stoff entsprechend dem endoskopischen Instrument (12) vorgesehen ist.

21. Endoskopisches Instrumentarium nach Anspruch 20, **dadurch gekennzeichnet, daß** das Manipulationsinstrument ein Trokar (44) ist.

22. Endoskopisches Instrumentarium nach Anspruch 18 oder 19, **dadurch gekennzeichnet, daß** das Beobachtungsinstrument ein Endoskop (14) ist, das mit einer endoskopischen Kamera (54) versehen ist.

23. Endoskopisches Instrumentarium nach Anspruch 22, **dadurch gekennzeichnet, daß** der endoskopischen Kamera (54) ein Bildverarbeitungssystem (56) nachgeschaltet ist, das die fluoreszierenden Markierungen im endoskopischen Bild laufend detektiert.

24. Endoskopisches Instrumentarium nach einem der Ansprüche 22 oder 23, **dadurch gekennzeichnet, daß** die Lichtquelle (48) zumindest im spektralen Anregungsbereich des fluoreszierenden Stoffes gepulstes Licht abgibt, und daß die Pulsfrequenz einer Videobildfrequenz der endoskopischen Kamera (54) entspricht.

25. Endoskopisches Instrumentarium nach einem der Ansprüche 18 bis 24, **dadurch gekennzeichnet, daß** das Beobachtungsinstrument distalseitig ein transparentes Element mit einem fluoreszierenden Stoff aufweist.

## Revendications

1. Instrument endoscopique pour réaliser des examens ou des interventions endoscopiques, comprenant une tige (16) munie d'une poignée (18) à l'extrémité proximale de la tige (16), et comprenant au moins un élément de travail (20), disposé à l'extrémité distale de la tige (16), au moins un marquage (26, 28, 30) étant prévu dans la zone de l'extrémité distale de l'instrument (12), et présentant une substance susceptible d'être excitée par une source lumineuse (48), **caractérisé en ce que** la substance est susceptible d'être excitée pour devenir fluorescente, et **en ce que** la substance fluorescente est sélectionnée de telle sorte que sa plage d'excitation se situe à l'intérieur de la plage d'excitation d'une autofluorescence de tissu ou dans la plage d'excitation d'un photosensibilisateur spécifique d'une tumeur.

2. Instrument endoscopique selon la revendication 1, **caractérisé en ce que** le marquage (26, 28, 30) est conformé en élément de marquage (32, 34, 36) disposé sur l'instrument (12).

3. Instrument endoscopique selon la revendication 2, **caractérisé en ce que** l'élément de marquage (32, 34, 36) est disposé de façon détachable.

4. Instrument endoscopique selon l'une des revendications 1 à 3, **caractérisé en ce que** l'élément de travail distal (20), au nombre minimum d'un, est pourvu d'un marquage (28, 30).

5. Instrument endoscopique selon la revendication 4, **caractérisé en ce que** les éléments de travail (20) sont conformés en deux mâchoires (22, 24), qui sont chacune pourvues d'un marquage (28, 30).

6. Instrument endoscopique selon l'une des revendications 1 à 5, **caractérisé en ce qu'**est prévu, tant sur l'élément de travail (20), au nombre minimum d'un, que dans la zone de l'extrémité distale de la tige (16), respectivement un marquage (26, 28, 30).

7. Instrument endoscopique selon la revendication 6, **caractérisé en ce que** l'élément de marquage (30) est conformé en douille tubulaire (36) susceptible d'être enfilée sur une tige tubulaire (16).

8. Instrument endoscopique selon l'une des revendications 2 à 7, **caractérisé en ce que** l'élément de marquage présente un revêtement en verre transparent ou en matière synthétique transparente, qui recouvre la substance fluorescente.

9. Instrument endoscopique selon la revendication 2 ou 3,
**caractérisé en ce que** l'élément de marquage est susceptible d'être déposé dans le corps dans lequel se déroule l'intervention endoscopique, et est susceptible d'y être ancré.

10. Instrument endoscopique selon l'une des revendications 1 à 9, **caractérisé en ce que** la substance fluorescente est sélectionnée dans le groupe comprenant la fluorescéine, l'éosine, les porphyrines, le sulfure de cadmium, l'acide aminolévulinique, l'hydrochlorure d'acide aminolévulinique, l'acridineorange, la tétracycline, l'auramine, la rhodamine B, la rhodamine C, l'auramine-carbol-fuscine, le sulfate de bleu de Nil.

11. Instrument endoscopique selon l'une des revendications 1 à 10, **caractérisé en ce que** la substance fluorescente est sélectionnée de telle sorte qu'elle soit susceptible d'être excitée pour devenir fluorescente dans une plage de longueurs d'ondes de 200 nm à 900 nm.

12. Instrument endoscopique selon la revendication 11, **caractérisé en ce que** la substance fluorescente est sélectionnée de telle sorte qu'elle soit susceptible d'être excitée dans une plage de longueurs d'ondes de 320 nm à 380 nm.

13. Instrument endoscopique selon la revendication 1, **caractérisé en ce que** la substance fluorescente est susceptible d'être excitée pour devenir fluorescente dans la plage de 400 nm à 500 nm, la plage d'excitation d'une autofluorescence de tissu.

14. Instrument endoscopique selon la revendication 1, **caractérisé en ce que** la substance fluorescente est susceptible d'être excitée dans la plage de 370 nm à 440 nm, la plage d'excitation d'un photosensibilisateur spécifique d'une tumeur.

15. Instrument endoscopique selon l'une des revendications 1 à 14, **caractérisé en ce que** plusieurs marquages (26, 28, 30) sont prévus, avec différentes substances fluorescentes susceptibles d'être excitées.

16. Instrument endoscopique selon l'une des revendications 1 à 15, **caractérisé en ce que** sont prévus plusieurs marquages (26, 28, 30) contenant différentes concentrations de substances fluorescentes.

17. Instrument endoscopique selon la revendication 9, **caractérisé en ce que** le marquage (26, 28, 30) présente une substance fluorescente telle qu'elle corresponde à celle d'un élément de marquage déposé dans le corps.

18. Instrumentation endoscopique, contenant un instrument endoscopique (12) selon l'une des revendications 1 à 17, et contenant, en outre, un dispositif d'acheminement de lumière et un instrument d'observation endoscopique, qui est relié à une source de lumière (48) qui est sélectionnée de telle sorte que la substance fluorescente soit susceptible d'être excitée pour devenir fluorescente.

19. Instrumentation endoscopique selon la revendication 18, **caractérisé en ce que** l'instrumentation endoscopique est un endoscope (14).

20. Instrumentation endoscopique selon la revendication 18 ou 19, **caractérisé en ce qu'**est prévu au moins un instrument de manipulation endoscopique, au moyen duquel l'instrument d'observation peut être introduit dans le corps et **en ce qu'**est prévu, sur la face interne de l'instrument de manipulation, au moins un marquage portant une substance fluorescente correspondant à l'instrument endoscopique (12).

21. Instrumentation endoscopique selon la revendication 20, **caractérisé en ce que** l'instrument de manipulation est un trocart (44).

22. Instrumentation endoscopique selon la revendication 18 ou 19, **caractérisé en ce que** l'instrument d'observation est un endoscope (14) équipé d'une caméra endoscopique (54).

23. Instrumentation endoscopique selon la revendication 22, **caractérisé en ce qu'**en aval de la caméra endoscopique (54) est monté un système de traitement d'images (56) qui détecte en continu les marquages fluorescents dans l'image endoscopique.

24. Instrumentation endoscopique selon l'une des revendications 22 ou 23, **caractérisé en ce que** la source de lumière (48) émet de la lumière pulsée au moins dans la plage spectrale d'excitation de la substance fluorescente, et **en ce que** la fréquence de répétition des impulsions correspond à une vidéofréquence de la caméra endoscopique (54).

25. Instrumentation endoscopique selon l'une des revendications 18 à 24; **caractérisé en ce que** l'instrument d'observation, du côté distal, présente un élément transparent revêtu d'une substance fluorescente.

## Claims

1. Endoscopic instrument for performing endoscopic examinations or procedures, having a shaft (16) having a handle (18) arranged at the proximal end of the shaft (16), and having at least one working element (20) arranged at the distal end of the shaft (16), wherein at least one marking (26, 28, 30), which has a substance that can be excited by a light source (48) is provided at the distal end region of the instrument, **characterized in that** the substance can be excited to fluoresce, and **in that** the fluorescing substance is selected in such a way that its excitation region lays in the excitation region of a tissue auto fluorescence or in the excitation region of a tumor-specific photosensitizer.

2. Endoscopic instrument of Claim 1, **characterized in that** the marking (26, 28, 30) is configured as a marking element (32, 34, 36) applied on the instrument (12).

3. Endoscopic instrument of Claim 2, **characterized in that** the marking element (32, 34, 36) is applied removably

4. Endoscopic instrument of anyone of Claims 1 through 3, **characterized in that** the at least one distal working element (20) is equipped with a marking (28, 30).

5. Endoscopic instrument of Claim 4, **characterized in that** the working elements (20) are configured as two mouth parts (22, 24) that are each equipped with a marking (28, 30).

6. Endoscopic instrument of anyone of Claims 1 through 5, **characterized in that** a marking (26, 28, 30) is respectively provided both on the at least one working element (20) and in the proximal end region of the shaft (16).

7. Endoscopic instrument of Claim 6, **characterized in that** the marking element (30) is configured as a tubular bushing (36) that can be slid onto a tubular shaft (16).

8. Endoscopic instrument of anyone of Claims 2 through 7, **characterized in that** the marking element has a coating, made of transparent glass or a transparent plastic, that covers the fluorescing substance.

9. Endoscopic instrument of Claims 2 or 3, wherein the marking element can be inserted into the body on which the endoscopic procedure is being performed, and can be anchored there.

10. Endoscopic instrument of anyone of Claims 1 through 9, **characterized in that** the fluorescing substance is selected from the group comprising fluorescein, eosin, the porphyrins, cadmium sulfide, aminolevulinic acid, aminolevulinic acid hydrochloride, Acridine Orange, tetracyclines, auramine, rhodamine B, rhodamine G, auramine Carbol Fuchsin, and Nile Blue sulfate.

11. Endoscopic instrument of anyone of Claims 1 through 10, **characterized in that** the fluorescing substance is selected so that it can be excited to fluoresce in the wavelength region from 200 to 900 nm.

12. Endoscopic instrument of Claim 11, **characterized in that** the fluorescing substance is selected so that it can be excited to fluoresce in the wavelength region from 320 to 380 nm.

13. Endoscopic instrument of Claim 1, **characterized in that** the fluorescing substance can be excited to fluoresce in the region from 400 nm to 500 nm which is an excitation region of a tissue auto fluorescence.

14. Endoscopic instrument of Claim 1, **characterized in that** the fluorescing substance can be excited in the region from 370 nm to 440 nm which is an excitation region of a tumor-specific photosensitizer.

15. Endoscopic instrument of anyone of Claims 1 through 14, **characterized in that** multiple markings (26, 28, 30) with differently excitable fluorescing substances are provided.

16. Endoscopic instrument of anyone of Claims 1 through 15, **characterized in that** multiple markings (26, 28, 30), which contain different concentrations of fluorescing substances, are present.

17. Endoscopic instrument of Claim 9, **characterized in that** the marking (26, 28, 30) has a fluorescing substance corresponding to that of a marking element inserted into the body.

18. Endoscopic instrument suite containing an endoscopic instrument (12) as defined in anyone of Claims 1 through 17, and further containing a light-supplying apparatus and an endoscopic observation instrument that is connected to light source (48) selected in such a way that the fluorescing substance can be excited to fluoresce.

19. Endoscopic instrument suite of Claim 18, **characterized in that** the endoscopic instrument suite is an endoscope (14).

20. Endoscopic instrument suite of Claims 18 or 19, **characterized in that** at least one endoscopic manipulation instrument is provided, through which the observation instrument can be introduced into the body; and **in that** at least one marking with a fluorescing substance corresponding to the endoscopic instrument (12) is provided on the inner side of the manipulation instrument.

21. Endoscopic instrument suite of Claim 20, **characterized in that** the manipulation instrument is a trocar (44).

22. Endoscopic instrument suite of Claims 18 or 19, **characterized in that** the observation instrument is an endoscope (14) that is equipped with an endoscopic camera (54).

23. Endoscopic instrument suite of Claim 22, **characterized in that** there is provided downstream from the endoscopic camera (54) an image processing system (56) that continuously detects the fluorescing markings in the endoscopic image.

24. Endoscopic instrument suite of Claims 22 or 23, **characterized in that** the light source (48) emits pulsed light at least in the spectral excitation region of the fluorescing substance; and **in that** the pulse frequency corresponds to a video image frequency of the endoscopic camera (54).

25. Endoscopic instrument suite of anyone of Claims 18 through 24, **characterized in that** the observation instrument has, at the distal end, a transparent element having a fluorescing substance.
